(19) Europäisches Patentamt
European Patent Office
Office européen des brevets

(11) **EP 4 528 750 A1**

(12) **EUROPEAN PATENT APPLICATION**

(43) Date of publication:
26.03.2025 Bulletin 2025/13

(21) Application number: 23213617.6

(22) Date of filing: 01.12.2023

(51) International Patent Classification (IPC):
**G16H 50/20** (2018.01) **G16H 50/30** (2018.01)
**G16H 50/70** (2018.01)

(52) Cooperative Patent Classification (CPC):
**G16H 50/20; G16H 50/30; G16H 50/70;**
G16H 10/40; G16H 30/40; G16H 40/63; G16H 40/67

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 25.09.2023 PCT/CN2023/202312

(71) Applicant: **Koninklijke Philips N.V.**
**5656 AG Eindhoven (NL)**

(72) Inventors:
• **JIANG, Zeyu**
**Eindhoven (NL)**
• **TIAN, Cong**
**Eindhoven (NL)**
• **FU, Yong**
**5656AG Eindhoven (NL)**
• **YUAN, yana**
**Eindhoven (NL)**

(74) Representative: **Philips Intellectual Property & Standards**
**High Tech Campus 52**
**5656 AG Eindhoven (NL)**

(54) **DETECTION OF HEMODYNAMIC INSTABILITY**

(57) Embodiments of the present disclosure relate to a method of detecting hemodynamic instability. The method includes obtaining a plurality of clinical parameters of a subject at a time point. Then, a plurality of prediction probabilities may be determined based on the plurality of clinical parameters, where each of the plurality of prediction probabilities is determined by a corresponding submodel of a plurality of sub-models in the machine learning model. The method also includes determining a combined prediction probability for the time point based on the plurality of prediction probabilities. The determined combined prediction probability is used to detect the hemodynamic instability. In accordance with embodiments of the present disclosure, the hemodynamic instability can be timely and precisely detected to alarm clinicians, thereby greatly reducing mortality and medical costs.

**FIG. 5**

**Description**

FIELD OF THE INVENTION

Embodiments of the present disclosure generally relate to a field of data processing, and more specially, to the detection of hemodynamic instability.

BACKGROUND OF THE INVENTION

Hemodynamic instability describes an unstable movement of blood. It is a condition or state in which a person's cardiovascular functions become unreliable, insufficient, or otherwise problematic. Therefore, hemodynamic instability is a crucial and common condition in the intensive care unit (ICU). Because the hemodynamic instability in post-traumatic patients cannot be detected timely, one third of ICU patients will develop shock with a higher mortality rate.

An early warning system of hemodynamic instability has the potential to improve the timely detection and initiation of intervention. If the hemodynamic instability of post-traumatic patients can be predicted timely, preventive treatment plans can be provided for high-risk patients, thereby greatly reducing mortality and medical costs. However, there are still some problems in the early warning system of hemodynamic instability, which needs to be solved to improve the predictions for hemodynamic instability.

SUMMARY OF THE INVENTION

In an early warning system of hemodynamic instability, a single-parameter shock indicator, (e.g., systolic blood pressure (SBP), and a shock index (heart rate/SBP)), is reported to detect hemodynamic instability. However, the patient condition will deteriorate in a later stage of shock and/or the risk is underestimated by only addressing cardiovascular system changes. Low-precision early warning systems are also not conducive to clinicians for identifying and interpreting relevant information, and thus it is difficult for clinicians to continuously supervise or evaluate ICU patients. A low-frequency and low-precision early warning system is not suitable for patients with acute changes in their condition. In the absence of predictive tools with sufficient accuracy, clinicians may resort to subjective judgment, which increases the risk that clinicians will act incorrectly.

In view of the above, embodiments of the present disclosure propose a method for detecting hemodynamic instability, a method of training a machine learning model for detecting hemodynamic instability, an electronic device, and a computer readable medium. The present disclosure can timely and precisely detect hemodynamic instability to alarm clinicians, thereby greatly reducing mortality and medical costs.

In a first aspect, the embodiments of the present disclosure provide a method of detecting hemodynamic instability. The method comprises obtaining a plurality of clinical parameters of a subject at a time point, the plurality of clinical parameters including at least two of vital sign data, lab measurement data, waveform feature data, and image data. The method further comprises determining, by a machine learning model, a plurality of prediction probabilities based on the plurality of clinical parameters, each of the plurality of prediction probabilities being determined by a corresponding sub-model of a plurality of sub-models in the machine learning model. The method further comprises determining a combined prediction probability for the time point based on the plurality of prediction probabilities. The method further comprises detecting the hemodynamic instability based on the combined prediction probability.

According to the embodiments of the present disclosure, the method uses at least two types of clinical parameters. Therefore, more parameters indicating the underlying pathophysiology of a cardiovascular system would be used to improve the prediction precision. Moreover, a plurality of clinical parameters may be used by a plurality of sub-models to determine a plurality of prediction probabilities and the combined prediction probability is determined from the plurality of prediction probabilities. The combined prediction probability would be more accurate, and thus the method can timely and precisely detect hemodynamic instability to alarm clinicians, thereby greatly reducing mortality and medical costs.

In some embodiments of the first aspect, the vital sign data includes at least one of a hearth rate, a temperature, a respiration rate, and a blood pressure, the lab measurement data includes at least one of creatinine, blood sugar, glutamic acid, lactic acid, alanine transaminase, and aspartate transaminase, the waveform feature data includes at least one of a peak-to-trough slope of a heart rate waveform, spacing between peaks of a heart rate waveform, a peak-to-trough slope of a ventilator waveform, and spacing between peaks of a ventilator waveform, and/or the image data includes at least one of an X-ray image, an ultrasound image, and a computed tomography image. In this way, multiple types of parameters are used to detect the hemodynamic instability to improve the prediction precision.

In some embodiments of the first aspect, obtaining a plurality of clinical parameters of a subject at a time point comprises determining whether a clinical parameter of the plurality of clinical parameters is null. Obtaining a plurality of clinical parameters of a subject at a time point further comprises in response to determining that the clinical parameter of the plurality of clinical parameters is null, determining whether latest data for the clinical parameter exists in a first filter time limit. Obtaining a plurality of clinical parameters of a subject at a time point further comprises in response to determining that the latest data for the clinical parameter exists in the first filter time limit, updating the clinical parameter based on the latest data. In this way, effective data of a clinical parameter of the plurality of clinical parameters can be obtained accurately even if the clinical parameter is null at current time point. For example, if the clinical parameter is null at the current time point and there is latest data for the clinical parameter in the filter time limit (for example, 2 hours), the latest data can be used. Therefore, this operation can improve the accurateness of the detection.

In some embodiments of the first aspect, obtaining a plurality of clinical parameters of a subject at a time point further comprises determining whether a further clinical parameter of the plurality of clinical parameters is null. Obtaining a plurality of clinical parameters of a subject at a time point further comprises in response to determining that the further clinical parameter of the plurality of clinical parameters is null, determining whether further latest data for the further clinical parameter exists in a second filter time limit, the second filter time limit being different from the first filter time limit. Obtaining a plurality of clinical parameters of a subject at a time point further comprises in response to determining that the further latest data for the further clinical parameter exists in the second filter time limit, updating the further clinical parameter based on the further latest data. For different types of clinical parameters, different filter time limits may be used. Therefore, the more effective data for the clinical parameters are used to improve the accurateness of the detection.

In some embodiments of the first aspect, the machine learning model is one of a plurality of machine learning models, the plurality of machine learning models form a detection model for detecting the hemodynamic instability, and each of the plurality of machine learning models is used to determine a combined prediction probability at a corresponding time point of a plurality of time points. In this way, for each of the plurality of time points, a corresponding machine learning model is used to detect the hemodynamic instability. Therefore, the accuracy for the hemodynamic instability detection is improved.

In some embodiments of the first aspect, determining a combined prediction probability for the time point based on the plurality of prediction probabilities comprises for each corresponding time point of the plurality of time points, determining a combined prediction probability by using a machine learning model respectively corresponding to the each corresponding time point based on the plurality of clinical parameters of the subject obtained at the each corresponding time point to obtain a plurality of combined prediction probabilities. Moreover, detecting the hemodynamic instability based on the combined prediction probability comprises selecting a predetermined number of combined prediction probabilities from the plurality of combined prediction probabilities, each selected combined prediction probabilities being greater than the remaining combined prediction probabilities of the plurality of combined prediction probabilities. Detecting the hemodynamic instability based on the combined prediction probability further comprises determining whether each of the predetermined number of combined prediction probabilities is greater than a predetermined probability threshold. Detecting the hemodynamic instability based on the combined prediction probability further comprises in response to determining that each of the predetermined number of combined prediction probabilities is greater than the predetermined probability threshold, determining that the hemodynamic instability occurs. In this way, the detection result of the hemodynamic instability can be more precise by utilizing a predetermined number of combined prediction probabilities.

In some embodiments of the first aspect, determining a predetermined number of combined prediction probabilities from the plurality of combined prediction probabilities comprises determining a predetermined ratio related to a model performance of the detection model based on at least one of: an accuracy rate, a precision rate, a recall rate, a specificity rate, an F1 score, and a false positive rate of the detection model. Determining a predetermined number of combined prediction probabilities from the plurality of combined prediction probabilities further comprises determining the predetermined number based on the predetermined ratio and the number of the plurality of machine learning models. In this way, the predetermined number of combined prediction probabilities can be obtained for providing the best model performance.

In some embodiments of the first aspect, detecting the hemodynamic instability based on the combined prediction probability comprises obtaining a plurality of clinical parameters of the subject for each of at least two further time points different from the time point. Detecting the hemodynamic instability based on the combined prediction probability further comprises for the each of at least two further time points, determining a combined prediction probability, by using a machine learning model respectively corresponding to the each of at least two further time points based on the plurality of clinical parameters obtained at the each of at least further time points. Detecting the hemodynamic instability based on the combined prediction probability further comprises detecting the hemodynamic instability based on the combined prediction probability for the time point and the combined prediction probabilities respectively for the at least two further time points. In this way, the hemodynamic instability can be accurately computed according to the clinical parameters at different time points.

In some embodiments of the first aspect, determining a combined prediction probability for the time point based on the plurality of prediction probabilities comprises determining an average probability of the plurality of prediction probabilities as the combined prediction probability. In this way, the combined prediction probability can be obtained more accurately.

In a second aspect, the embodiments of the present disclosure provide a method of training a machine learning model for detecting hemodynamic instability. The method comprises obtaining a plurality of sample clinical parameters of a plurality of sample subjects at a time point, the plurality of sample clinical parameters including at least two of vital sign data, lab measurement data, waveform feature data, and image data. The method further comprises determining a ratio of a number of positive samples in the plurality of sample subjects to a number of negative samples in the plurality of sample subjects. The method further comprises generating a plurality of sub-datasets, each sub-dataset including sample clinical parameters corresponding to the positive samples and sample clinical parameters corresponding to one portion of the negative samples, wherein the negative samples are divided into a number of portions based on the ratio, and the one portion of the negative samples for the each sub-dataset is different from each other. The method further comprises training a plurality of sub-models in the machine learning model for detecting the hemodynamic instability based on the plurality of sub-datasets.

According to the embodiments of the present disclosure, the method uses at least two types of sample clinical parameters. Therefore, more sample clinical parameters indicating the underlying pathophysiology of cardiovascular system would be used to improve the precision. Moreover, the positive samples and the negative samples are balanced to form a plurality of sub-datasets. The plurality of sub-datasets may be used to train a plurality of sub-models in the machine learning model. Because the balanced sub-datasets are used during the training, the precision for each sub-model can be improved. Thereby, the trained machine learning model can detect the hemodynamic instability accurately.

In some embodiments of the second aspect, the machine learning model is one of a plurality of machine learning models, the plurality of machine learning models form a detection model for detecting the hemodynamic instability, and the method further comprises obtaining a further plurality of sample clinical parameters of the plurality of sample subjects at a further time point different from the time point. The method further comprises training a further machine learning model of the plurality of machine learning models based on the further plurality of sample clinical parameters. In this way, the sample clinical parameters of the plurality of sample subjects at different time points are used to train different machine learning models, thereby improving the performance of the detection model.

In some embodiments of the second aspect, obtaining a plurality of sample clinical parameters of a plurality of sample subjects at a time point comprises determining whether a sample clinical parameter of the plurality of sample clinical parameters is null. Obtaining a plurality of sample clinical parameters of a plurality of sample subjects at a time point further comprises in response to determining that the sample clinical parameter of the plurality of sample clinical parameters is null, determining whether latest data for the sample clinical parameter exists in a first filter time limit. Obtaining a plurality of sample clinical parameters of a plurality of sample subjects at a time point further comprises in response to determining that the latest data for the sample clinical parameter exists in the first filter time limit, updating the sample clinical parameter based on the latest data. In this way, effective data of the clinical parameter of the plurality of clinical parameters can be obtained accurately even if the value of the clinical parameter is null at current time point, thereby improving the prediction result.

In some embodiments of the second aspect, training a plurality of sub-models in the machine learning model for detecting the hemodynamic instability based on the plurality of sub-datasets comprises obtaining a prediction probability of a sub-model of the plurality of sub-models by inputting a sub-dataset of the plurality of sub-datasets into the sub-model. Training a plurality of sub-models in the machine learning model for detecting the hemodynamic instability based on the plurality of sub-datasets further comprises adjusting model parameters of the sub-model based on the prediction probability and a real result of hemodynamic instability. In this way, the trained sub-models are more precise and accurate.

In a third aspect, the embodiments of the present disclosure provide an electronic device, comprising: at least one processor; and at least one memory having a plurality of instructions stored thereon, when executed by the at least one processor, cause the device to perform the method of the first aspect or the second aspect.

In a fourth aspect, the embodiments of the present disclosure provide a computer readable medium having computer instructions stored thereon, the computer instructions, when executed a processor, causing the processor to perform the method of the first aspect or the second aspect.

It is to be understood that the Summary is not intended to identify key or essential features of embodiments of the present disclosure, nor is it intended to be used to limit the scope of the present disclosure. Other features of the present disclosure will become easily comprehensible through the description below.

BRIEF DESCRIPTION OF THE DRAWINGS

The details of one or more embodiments of the present disclosure are set forth in the accompanying drawings and the description below. Other features, aspects, and advantages of the disclosure will become apparent from the description, the drawings, and the claims, wherein:

Fig. 1 is a block diagram of an example environment in which example embodiments of the present disclosure may be implemented;

Fig. 2 is a schematic diagram illustrating a model structure for detecting hemodynamic instability in accordance with example embodiments of the present disclosure;

Fig. 3 is a flowchart illustrating an example process for detecting hemodynamic instability in accordance with example embodiments of the present disclosure;

Fig. 4 is a table illustrating example clinical parameters in accordance with example embodiments of the present disclosure;

Fig. 5 is a flowchart illustrating an example method for detecting hemodynamic instability in accordance with example embodiments of the present disclosure;

Fig. 6 is a block diagram of an example environment in which a machine learning model for detecting hemodynamic instability is trained in accordance with example embodiments of the present disclosure;

Fig. 7 is a block diagram illustrating a model structure for detecting hemodynamic instability in accordance with example embodiments of the present disclosure;

Fig. 8 is a schematic diagram illustrating an example process for training a machine learning model in accordance with example embodiments of the present disclosure;

Fig. 9 is a flowchart illustrating an example method for training a machine learning model for detecting hemodynamic instability in accordance with example embodiments of the present disclosure;

Fig. 10A, 10B, and 10C are tables illustrating model performances of a model for detecting hemodynamic instability in accordance with example embodiments of the present disclosure; and

Fig. 11 is a schematic diagram illustrating example devices suitable for implementing embodiments of the present disclosure.

Throughout the figures, same or similar reference numbers will always indicate same or similar elements.

DETAILED DESCRIPTION OF EMBODIMENTS

Principle of the present disclosure will now be described with reference to some example embodiments. It is to be understood that these embodiments are described only for the purpose of illustration and help those skilled in the art to understand and implement the present disclosure, without suggesting any limitations as to the scope of the disclosure. The disclosure described herein can be implemented in various manners other than the ones describe below.

As used herein, the term "comprise/include" and its variants are to be read as open terms that mean "comprise/include, but not limited to." The term "based on" is to be read as "based at least in part on." The term "one embodiment" and "an embodiment" are to be read as "at least one embodiment." The term "another embodiment" is to be read as "at least one other embodiment." Moreover, it is to be understood that in the context of the present disclosure, the terms "first," "second" and the like are used to indicate individual elements or components, without suggesting any limitation as to the order of these elements. Further, a first element may or may not be the same as a second element.

As mentioned above, when the single-parameter shock indicator is used to detect hemodynamic instability, the early warning systems have a low-precision and it is difficult for clinicians to continuously supervise or evaluate ICU patients. The low-frequency and low-precision early warning systems will be not suitable for patients with acute changes in their conditions. Moreover, different kinds of clinical parameters (e.g., the vital signs, physical examination, and laboratory measurements) may be used to identify the hemodynamic instability. Clinicians in ICUs will obtain a large amount of the above clinical parameters from multiple monitoring systems and devices. However, the limited ability of humans to process complex information alone hinders the early identification of patient deterioration. Therefore, conventional identification and early warning methods are difficult to monitor patients' conditions in real time.

To address these and other potential problems, embodiments of the present disclosure provide a solution for detecting hemodynamic instability. More parameters indicating the hemodynamic instability are used to improve the prediction precision. Moreover, a plurality of clinical parameters would be used by a plurality of sub-models to determine a plurality of prediction probabilities and the combined prediction probability is determined from the plurality of prediction probabilities. The combined prediction probability would be more accurate, and thus the hemodynamic instability can be detected precisely and timely, thereby greatly reducing mortality and medical costs.

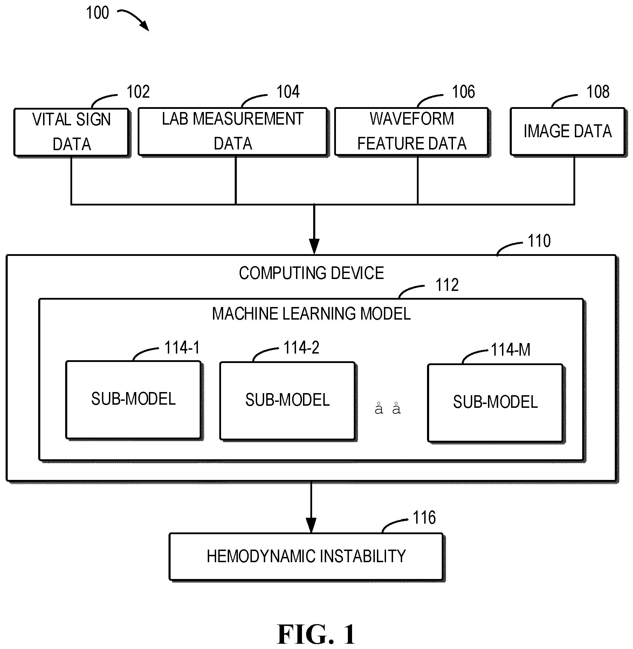

Reference is now made to Fig. 1 which illustrates an example environment in which example implementations of the present disclosure may be implemented. The example environment includes a computing device 110. The computing device 110 may be used to detect hemodynamic instability based on certain clinical parameters. Example of the computing device 110 includes, but is not limited to, a personal computer, a server computer, a handheld or laptop device, a mobile device, a multiprocessor system, consumer electronics, a minicomputer, a mainframe computer, and a distributed computing environment including any of the above systems or devices.

As shown in Fig. 1, the computing device 110 may receive a plurality of clinical parameters for a subject at various time points, a patient in a hospital, for example. In some embodiments, the plurality of clinical parameters includes at least two of vital sign data 102, lab measurement data 104, waveform feature data 106, and image data 108.

The vital sign data 102 is measurement results of the body's most basic functions, and includes at least one of a heart rate (Hr), a temperature, a respiration rate, a blood pressure and so on. The lab measurement data 104 is obtained from the lab, and it includes at least one of creatinine, blood sugar, glutamic acid, lactic acid, alanine transaminase, aspartate transaminase, and so on. The waveform feature data 106 includes at least one of a peak-to-trough slope of a heart rate waveform, spacing between peaks of a heart rate waveform, a peak-to-trough slope of a ventilator waveform, spacing between peaks of a ventilator waveform, and so on. The image data 108 includes at least one of an X-ray image, an ultrasound image, a computed tomography image, and so on.

After receiving the plurality of clinical parameters corresponding to a time point, the computing device 110 may use the machine learning model 112 to detect hemodynamic instability. The machine learning model 112 includes a sub-model 114-1, a sub-model 114-2, ..., and a sub-model 114-M, where M is an integer. For convenience, the sub-models 114-1, 114-2, ..., and 114-M are collectively referred as sub-models 114. Each of the sub-models 114 is used to generate a prediction probability based on the plurality of clinical parameters. The prediction probabilities of the sub-models 114 are used to determine a combined prediction probability. The sub-models 114 may be implemented using machine models. For example, the sub-models 114 may be random forests, extreme gradient boosting, convolutional neural networks (CNNs), recurrent neural networks (RNNs), long short-term memories (LSTMs), LSTMs + Attention, CNN + bidirectional long short-term memory (BiLSTM) + Attention, etc. In some embodiments, different kinds of machine models are trained by using the same training data. The machine model which has the best training results is selected from the different kinds of machine models and used as the sub-model. In some embodiments, the sub-models are the same kind of machine models. In some embodiment, the sub-models are different kinds of machine models.

The computing device 110 may further determine whether there is hemodynamic instability 116 or not based on the combined prediction probability of the machine learning model 112. Fig. 1 shows an example that the computing device 110 includes a machine learning model 112. In some embodiments, the computing device 110 includes a plurality of machine learning models to generate a plurality of combined prediction probabilities and the computing device 110 uses the plurality of combined prediction probabilities to alarm the hemodynamic instabilities.

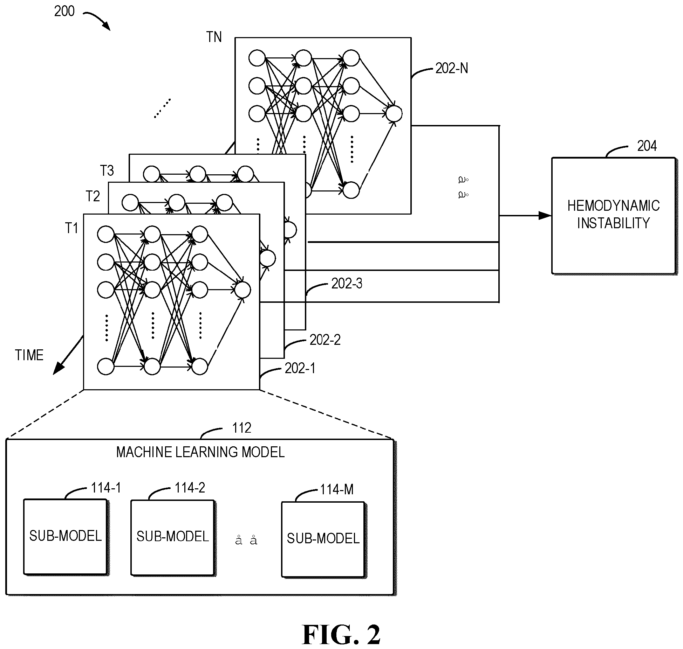

Reference is now made to Fig. 2 which illustrates a model structure 200 for detecting hemodynamic instability in accordance with example embodiments of the present disclosure. In Fig.2, a machine learning model 202-1, a machine learning model 202-2, a machine learning model 202-3, ..., and a machine learning model 202-N form a detect model which is used to detect hemodynamic instability 204, where N is an integer. For convenience, the machine learning model 202-1, 202-2, 202-3, ..., and 202-N are collectively referred as machine learning models 202.

As shown in Fig. 2, the machine learning model 202-1 may be the same as the machine learning model 112 shown in Fig. 1, which includes a plurality of sub-model 114-1, sub-model 114-2, ..., and sub-model 114-M. Additionally, each of machine learning models 202 has the same model structures and corresponds to a different time point. For example, the machine learning model 202-1 corresponds to a time point T1, the machine learning model 202-2 corresponds to a time point T2, the machine learning model 202-3 corresponds to a time point T3, ..., the machine learning model 202-N corresponds to a time point IN. For example, there may be 24 machine learning models and each of the 24 machine learning models corresponds to a time length of one hour. Although the structures of the machine learning models 202 are the same, each of the machine learning models 202 is trained using the sample clinical parameters of a plurality of sample subjects at a different time point. Therefore, each of the machine learning models 202 may generate a combined prediction probability at a corresponding time point. In this case, the machine learning models 202 may generate a plurality of combined prediction probabilities, which are used to detect the hemodynamic instability 204. If the hemodynamic instability 204 is detected, an alarm may be generated to inform the clinicians. Then the clinicians may provide preventive treatment plans for the subject or the patient.

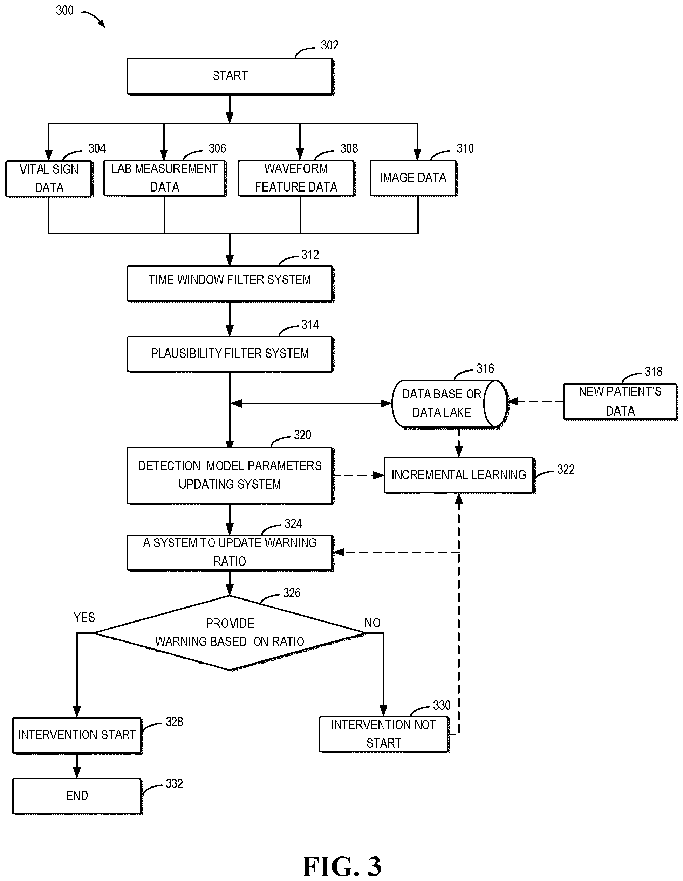

Reference is now made to Fig. 3 which illustrates an example process 300 for detecting hemodynamic instability in accordance with example embodiments of the present disclosure.

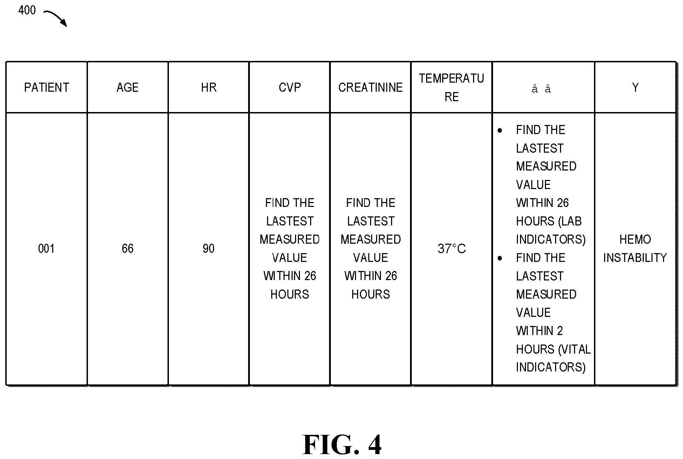

The process 300 starts at block 302. Then, the vital sign data 304, lab measurement data 306, waveform feature data 308, and image data 310 for one subject are collected by multiple data collection systems. All collected data is entered into the time window filter system 312 in a rolling manner with a certain time window (the default time window may be 1 hour, but it may be customized such as 2 hours, or 30 minutes, etc.). In some embodiments, if a parameter is null, the hemodynamic instability may be detected by using other parameters without the information of this parameter. In some embodiments, if a parameter is null, the spare data for the parameter will be generated within a certain rolling time window. Rolling time imputation method will be set up in a plausibility filter system 314 to determine the spare data for the parameter. For example, Fig. 4 is a table illustrating example clinical parameters 400 in accordance with example embodiments of the present disclosure. As shown in Fig. 4, 001 patient has age, heart rate (hr), and temperature value in t0, but does not have values with central venous pressure (CVP) and creatinine. The system will filter values every hour before t0 to find the latest value as the spare data. The maximum filter time limit is 26 hours before t0 for lab testing indicators. For vital sign indicators, the maximum filter time limit is 2 hours. If the system finds the latest value, the parameter will be set as the latest value; and if no latest value is found, it will remain blank (or null).

Returning back to Fig. 3, the plausibility filter system 314 may further remove abnormal data from the collected data, for example, abnormal blood pressure of the subject. A detection model parameters updating system 320 is used to automatically compute the parameters of the hemodynamic instability model by training the hemodynamic instability model, and automatically calculate the probabilities of hemodynamic instability for each subject using the collected data. The detection model parameters updating system 320 may be implemented by the model structure 200 shown in Fig. 2. Subsequently, a warning threshold is obtained from a system 324 to update warning threshold. The system 324 is used to update the warning threshold. The results obtained from the detection model are compared with the warning threshold to determine whether to provide a warning at block 326. If it is determined to provide a warning, intervention starts at block 328. For example, the clinicians will provide preventive treatment plans for the subject. Then, this method ends at block 332. If it is determined not to provide a warning, the results obtained from the detection mode show that the patient is good and the intervention does not start at block 330.

As shown in Fig. 3, the solid line arrows indicate the normal working flow for detecting the hemodynamic instability. Additionally, the dotted arrows indicate that when new patients' data 308 is added to the database or the data lake 316, the parameters of the detection model may be updated. For example, when new patient's data 318 is added to the database or data lake 316. The new dataset in the database or data lake 316 can be used for incremental learning 322. During the incremental learning process, a positive sample indicates that a subject has the hemodynamic instability, and a negative sample indicates that a subject does not have the hemodynamic instability. In one embodiment, during the incremental learning process, if a positive sample cannot be detected correctly, this process continues until the positive sample can be detected correctly. Moreover, the warning threshold which is used to determine whether the patient has the hemodynamic instability may be adjusted. For example, the warning threshold is set as 0.5. When a prediction probability for a sample is greater than or equal to 0.5, the hemodynamic instability would be detected. When a prediction probability for a sample is less than 0.5, the hemodynamic instability would not be detected. During the actual training process, the prediction probability for a positive sample may be 0.45. If the warning threshold is set as 0.5, the positive sample cannot be detected. Therefore, the warning threshold may be adjusted as 0.4. At this time, the positive sample would be detected. If the computing device 110 monitors the changes in the medical environment in the area, for example, the presence of a large number of patients or a change in patient age, it uses incremental learning to update the parameters of the sub-models of the machine learning models of the detection model.

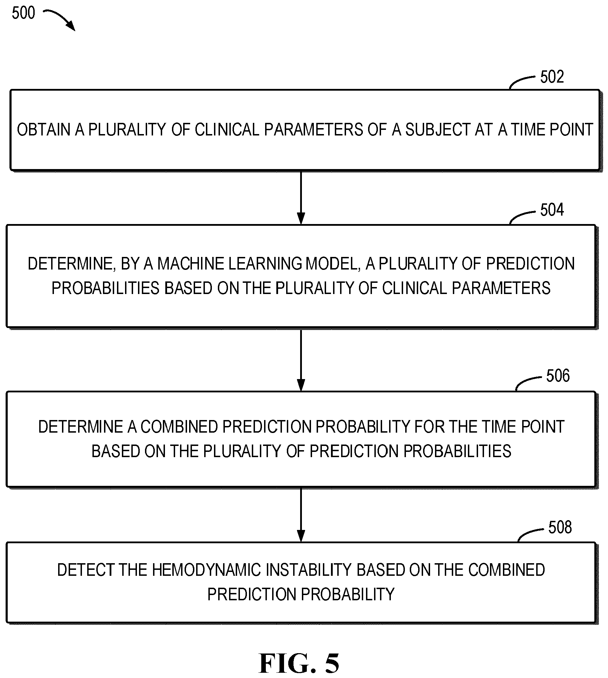

Fig. 5 is a flowchart illustrating an example method 500 for detecting hemodynamic instability in accordance with example embodiments of the present disclosure. The example process may be performed in the computing device 110 or any other suitable computing device.

At block 502, the computing device 110 obtains a plurality of clinical parameters of a subject at a time point. The plurality of clinical parameters may include at least two of vital sign data, lab measurement data, waveform feature data, and image data. The at least two of the vital sign data, the lab measurement data, the waveform feature data, and the image data are used to determine the hemodynamic instability of a subject, such as a patient.

In some embodiments, the computing device needs to check the plurality of clinical parameters of a subject at a time point. Therefore, the computing device 110 checks whether a clinical parameter of the plurality of clinical parameters is null. If the clinical parameter is not null, it will be used. Additionally, if the clinical parameter is null, it is also used to determine the hemodynamic instability or the hemodynamic instability can be determined without the consideration of this clinical parameter. Alternatively, if the computing device 110 determines that the clinical parameter of the plurality of clinical parameters is null, it will determine a filter time limit for the clinical parameter and find the latest data for the clinical parameter in the filter time limit. The filter time limit may be referred as a first filter time limit. If the computing device 110 finds the latest data for the clinical parameter in the filter time limit, then the first clinical parameter is updated as the latest data. If the computing device 110 cannot find latest data for the clinical parameter in the first filter time limit, then the clinical parameter will remain null and the process will continue.

In some embodiments, the computer device 110 also checks whether a further clinical parameter of the plurality of clinical parameters is null. The further clinical parameter is different from the clinical parameter. The computer device 110 determines that the further clinical parameter of the plurality of clinical parameters is null. If the further clinical parameter is not null, the further clinical parameter will be used. If the further clinical parameter is null, the computing device 110 determines a second filter time limit for the further clinical parameter and finds the latest data for the further clinical parameter in a second filter time limit, where the second filter time limit is different from the first filter time limit. If the computing device 110 finds the latest data for the further clinical parameter in the second filter time limit, the further clinical parameter is updated as the latest data. If the computing device 110 does not find the latest data for the further clinical parameter in the second filter time limit, the parameter will remain null and the process will continue. Additionally, the filter time limit for each of the plurality of clinical parameters may be different.

At block 504, the computing device 112 determines a plurality of prediction probabilities by a machine learning model based on the plurality of clinical parameters, where each of the plurality of prediction probabilities is determined by a corresponding sub-model of a plurality of sub-models included in the machine learning model. The plurality of sub-models may be pre-trained, and the training processes will be described later.

In some embodiments, the computing device 110 inputs the plurality of clinical parameters into each of the plurality of sub-models to obtain the plurality of prediction probabilities. For example, if there are three sub-models in the machine learning model, three prediction probabilities are generated. Moreover, the machine learning model is associated with a corresponding time point of a plurality of time points. Therefore, for the corresponding time point, the plurality of sub-models may output the plurality of prediction probabilities.

At block 506, the computing device 110 determines a combined prediction probability for the time point based on the plurality of prediction probabilities for the time point. The plurality of prediction probabilities obtained from the plurality of sub-models may be used to generate the combined prediction probability. In some embodiments, the computing device 110 computes an average probability of the plurality of prediction probabilities as the combined prediction probability. In some embodiments, the computing device 110 computes a weighted average probability of the plurality of prediction probabilities as the combined prediction probability. The above embodiments are used to illustrate the disclosure, rather than to limit the scope of the present disclosure.

At block 508, the computing device 110 detects the hemodynamic instability based on the combined prediction probability. In some embodiments, the combined prediction probability is used to determine the hemodynamic instability of the subject. In some embodiments, the combined prediction probability and some combined prediction probabilities of other machine learning models are used to determine the hemodynamic instability of the subject together.

In some embodiments, the machine learning model is one of a plurality of machine learning models. The plurality of machine learning models form a detection model for detecting the hemodynamic instability, and each of the plurality of machine learning models is used to determine a combined prediction probability at a corresponding time point of a plurality of time points. The combined prediction probability is determined based on the plurality of clinical parameters for the corresponding time point. Therefore, there may be a plurality of combined prediction probabilities, each of which corresponds to one of a plurality of time points, respectively. For example, if the period for detecting the hemodynamic instability is 24 hours, 24 machine learning models are used to detect the hemodynamic instability and each of 24 machine learning models corresponds to one hour of 24 hours. The data during the one hour is accessed by the corresponding machine learning model.

In some embodiments, for each corresponding time point of the plurality of time points, the computing device 110 uses the machine learning model respectively corresponding to the each corresponding time point of the plurality of time points to process the plurality of clinical parameters obtained at the each corresponding time point of the plurality of time points to generate a combined prediction probability for the each corresponding time point of the plurality of time points. Therefore, a plurality of combined prediction probabilities respectively for the plurality of time points may be obtained. In order to alarm the hemodynamic instability precisely, the computing device 110 first selects a predetermined number of combined prediction probabilities from the plurality of combined prediction probabilities. The selected combined prediction probabilities are greater than the remaining combined prediction probabilities of the plurality of combined prediction probabilities. Next, the computing device 110 determines whether each of the predetermined number of combined prediction probabilities is greater than a predetermined probability threshold. If each of the predetermined number of combined prediction probabilities is greater than the predetermined probability threshold, the computing device 110 determines that the hemodynamic instability occurs and an alarm is produced. If at least one of the predetermined number of combined prediction probabilities is less than or equal to the predetermined probability threshold, the computing device 110 determines that the hemodynamic instability does not occur. For example, the predetermined probability threshold may be set as 0.5 and the predetermined number is 16. The 16 combined prediction probabilities are selected from the plurality of combined prediction probabilities. If all of the 16 combined prediction probabilities are greater than 0.5, an alarm for the hemodynamic instability is generated. If at least one of the 16 combined prediction probabilities is less than or equal to the predetermined probability threshold, the hemodynamic instability will not occur.

In some embodiments, the predetermined number is the number of the plurality of machine learning models in the detection model. In this case, the computing device 110 determines that the hemodynamic instability occurs only if all the combined prediction probabilities of all the plurality of machine learning models are greater than the predetermined probability threshold. In some embodiments, the predetermined number is one. In this case, the computing device 110 determines that the hemodynamic instability occurs only if one of the plurality of combined prediction probabilities is greater than the predetermined probability threshold. For example, the computing device 110 may select a maximum combined prediction probability from the plurality of combined prediction probabilities and determines whether the maximum combined prediction probability is greater than the predetermined probability threshold.

In some embodiments, the predetermined number is determined by a predetermined ratio and the number of the plurality of machine learning models. The predetermined ratio reflects a model performance of the detection model and is determined based on at least one of: an accuracy rate, a precision rate, a recall rate, a specificity rate, an F1 score, and a false positive rate of the detection model. For example, the predetermined ratio may be set as 35% based at least on the accuracy rate and a recall rate. If the detection model includes 24 machine learning models, the predetermined number is $24*(1-35\%)\approx16$. The computing device 110 will determine whether each of the predetermined number of combined prediction probabilities is greater than a predetermined probability threshold. In one example, the computing devices ranks the plurality of combined prediction probabilities in descending order and determines whether the 16th combined

prediction probability is greater than the predetermined probability threshold. If the 16th combined prediction probability is greater than the predetermined probability threshold, it represents that each of the predetermined number of combined prediction probabilities is greater than a predetermined probability threshold and an alarm for the hemodynamic instability is generated. If the 16th combined prediction probability is less than or equal to the predetermined probability threshold, there is no alarm.

In some embodiments, the computing device 110 also obtains a plurality of clinical parameters of the subject for each of at least two further time points different from the time point. Then, for the each of at least two further time points, the computing device 110 uses a machine learning model respectively corresponding to the each of at least two further time points to process the plurality of clinical parameters obtained at the each of the at least two time points to generate a combined prediction probabilities of the hemodynamic instability for the each of the at least two time points. Then the computing device 110 detects the hemodynamic instability based on the combined prediction probability for the time point and the combined prediction probabilities respectively for the at least two further time points.

In some embodiments, the computing device 110 averages the first combined prediction probability at the time point and previous combined prediction probabilities at previous time points to determine a first average prediction probability. After the computing device 110 receives the second plurality of clinical parameters at a further time point and uses the further machine learning model of the plurality of machine learning models corresponding to the further time point to compute a second combined prediction probability of the hemodynamic instability. The further time point may be adjacent to the time point. Then the computing device 110 averages the second combined prediction probability, the first combined prediction probability and previous combined prediction probabilities to determine a second average prediction probability. The computing device 110 further computes the difference between the first average prediction probability and the second average prediction probability and determines whether the difference is great than a threshold. If the difference is greater than the threshold, the computing device 110 determines that there is hemodynamic instability for the subject at the further time point and generates an alarm. If the difference is less than or equal to the threshold, the computing device 110 will continue to detect the hemodynamic instability.

In some embodiments, the computing device 110 obtains the first combined prediction probability at the time point using the machine learning model corresponding to the time point. Then, the computing device 110 computes a first difference between the first combined prediction probability and a previous combined prediction probability at a previous adjacent time point. Then the computing device 110 computes a first average difference of the first difference and the previous differences. The previous differences are determined from combined prediction probabilities of any two adjacent time points of previous time points. For example, the time point is 5 o'clock. The computing device 110 computes the first difference by subtracting the previous combined prediction probability for 4 o'clock from the first combined prediction probability for 5 o'clock, the first previous difference by subtracting the combined prediction probability for 3 o'clock from the combined prediction probability for 4 o'clock, the second previous difference by subtracting the combined prediction probability for 2 o'clock from the combined prediction probability for 3 o'clock, and the third previous difference by subtracting the combined prediction probability for 1 o'clock from the combined prediction probability for 2 o'clock. Then the computing device computes the first average difference of the first difference, the first previous difference, the second previous difference, and the third previous difference. The computer device 110 may obtain a plurality clinical parameter at a next time point adjacent to the time point and use a machine learning model corresponding the next time point to obtain a second combined prediction probability. For example, if the time point is 5 o'clock, the next time point is 6 o'clock. The second average difference at the next time point is computed in a similar way. If the difference between the first average difference and the second average difference is greater than a threshold, the computing device 110 determines that there is hemodynamic instability for the subject at the next time point and generates an alarm. If the difference is less than or equal to the threshold, the computing device will continue to detect the hemodynamic instability.

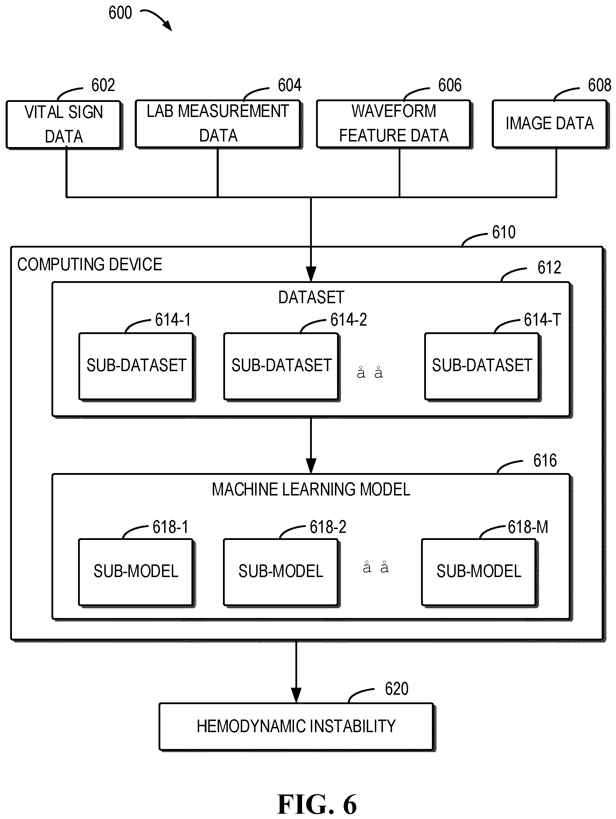

Reference is now made to Fig. 6, which illustrates a block diagram of an example environment in which a machine learning model for detecting hemodynamic instability is trained in accordance with example embodiments of the present disclosure. The example environment 600 includes a computing device 610. The computing device 610 can be used to train a machine learning model for detecting hemodynamic instability based on some clinical parameters. In some embodiments, the computing device 610 and the computing device 110 shown in Fig. 1 are the same device. In some embodiments, the computing device 610 and the computing device 110 shown in Fig. 1 are different devices.

As shown in Fig. 6, the computing device 610 may receive a plurality of sample clinical parameters for a plurality of sample subjects. In some embodiments, the plurality of sample clinical parameters includes at least two of vital sign data 602, lab measurement data 604, waveform feature data 606, and image data 608.

After receiving the plurality of clinical parameters of a plurality of sample subjects, the received plurality of clinical parameters are used as a dataset 612. As mentioned before, hemodynamic instability does not usually occur in the real world, and the positive cases (hemodynamically unstable patients) are not too much, and the negative cases (hemodynamically stable patients) are much more than the positive cases. The training data is an extremely imbalance data. If this extremely imbalance data is input into the training model, the final performance will extremely bad since the final predicted results will target to negative cases (hemodynamic stable patients) rather than positive cases (hemodynamic instability patients). Therefore, the new training framework that is specific for the imbalance data is provided. Therefore, the computing device 610 divides the dataset 612 into a plurality of sub-datasets, for example, a sub-dataset 614-1, a sub-dataset 614-2, ..., and a sub-dataset 614-T, where T is an integer. For convenience, the sub-datasets 614-1, 614-2, ..., and 614-T are collectively referred as sub-datasets 614. The plurality of sub-datasets can be balanced according to a ratio of positive cases/negative cases. For example, the dataset 612 includes a total of 3671 positive cases (hemodynamically unstable patients) and 14767 negative cases (hemodynamically stable patients), and the positive and negative ratio is close to 1:3. Therefore, 3 balanced sub-datasets are generated, and 3 sub-models are trained with the balanced sub-dataset, each sub-model with a predetermined number of cross validations. For example, the predetermined number is set as 5. Each of 3 sub-models has the same positive cases and one of different parts of the negative cases, and the number of the same positive cases is equal to the number of one of different parts of the negative cases.

The computing device 610 may use the plurality of sub-datasets to train the machine learning model 616 for detect hemodynamic instability. The machine learning models 616 includes a sub-model 618-1, a sub-model 618-2, ..., and a sub-model 618-M, where M is an integer. For convenience, the sub-models 618-1, 618-2, ..., and 618-M are collectively referred as sub-models 618. Each of the sub-models 618 is trained by at least one of the sub-datasets 614. During the training process, the parameters of each of the sub-models 618 is determined by comparing the prediction probability of each of the sub-models 618 with a real result of hemodynamic instability. Finally, these sub-models' prediction results are integrated together to form the combined prediction probability of the machine learning model.

The computing device 610 may also determine whether there is hemodynamic instability 620 based on the prediction probability of the machine learning model. Fig. 6 shows that the computing device 610 includes a machine learning model, which is used to describe the disclosure rather than limitations. In some embodiments, the computing device 610 includes a plurality of machine learning models that may generate a plurality of combined prediction probabilities. The computing device 610 uses these combined prediction probabilities to detect and alarm the hemodynamic instabilities.

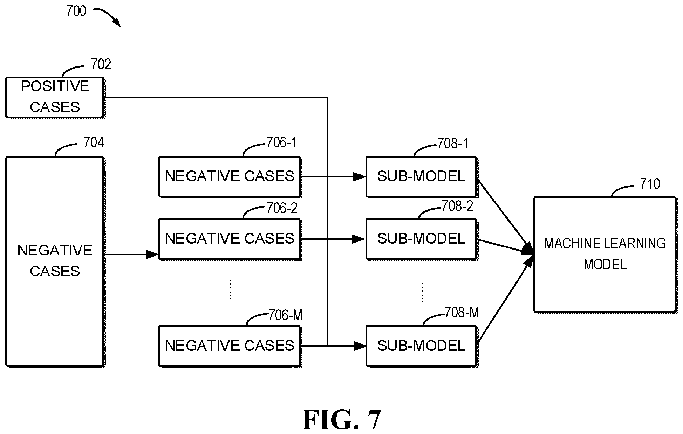

Reference is now made to Fig. 7, which illustrates a model structure for detecting hemodynamic instability in accordance with example embodiments of the present disclosure. There are fewer positive cases 702 and more negative cases 704 for the hemodynamic instability. Therefore, the numbers of the positive cases 702 and the negative cases 704 are unbalanced. Therefore, the negative cases 704 are divided into negative cases 706-1, negative cases 706-2, ..., and negative cases 706-M, which are different from each other. Then the same positive cases 702 are combined with each of negative cases 706-1, negative cases 706-2, ..., and negative cases 706-M to form sub-datasets to train sub-models. For, example, the positive cases 702 is combined with negative cases 706-1 for training a sub-model 708-1, the positive cases 702 is combined with negative cases 706-2 for training s sub-model 708-2, ..., and the positive cases 702 are combined with negative cases 706-M for training a sub-model 708-M, where M is an integer. During the training process, the parameters of the sub-models are updated. After being trained, the sub-models 708-1, 708-2, ..., and 708-M form the machine learning model 710. The output of the machine learning model is based on the outputs of the sub-models 708. For example, the plurality of prediction probabilities of the sub-models 708 may be averaged to generate the output of the machine learning model 710, such as the combined prediction probability. The number of the sub-models 708 may be determined based on the ratio of positive cases/negative cases. For example, if the ratio of positive cases/negative cases is 3, the number of the sub-models 708 may be 3.

For example, there are a total of 3671 hemodynamically unstable cases (positive) and 14767 hemodynamically stable cases (negative). The positive and negative ratio is close to 1:3, so the negative cases have been separated into 3 parts to pair with positive cases. Therefore, positive cases are repeated 3 times and paired with to each part of the negative cases respectively, then three sub-models can be trained. The parameters of each of the three models are updated by a corresponding residual error. After that, these three sub-models have been combined together. The first model is used as a classifier to predict result, the prediction result is compared with the ground truth, and the first residual error was generated. If this residual error is close to zero, it indicates the wonderful results. However, it could not be successful in the first model since it is a weak classifier. Therefore, the first model keeps case data with right predictions, and transfers case data related to incorrect predictions to a second model to get a lower residual error value. The remaining models may be done in the same manner until the lowest residual errors are obtained. For example, if a positive case cannot be correctly detected by the first model during the training process, this positive case may be used to train the second model. If the second model can detect the positive case correctly, the lower residual error is obtained。

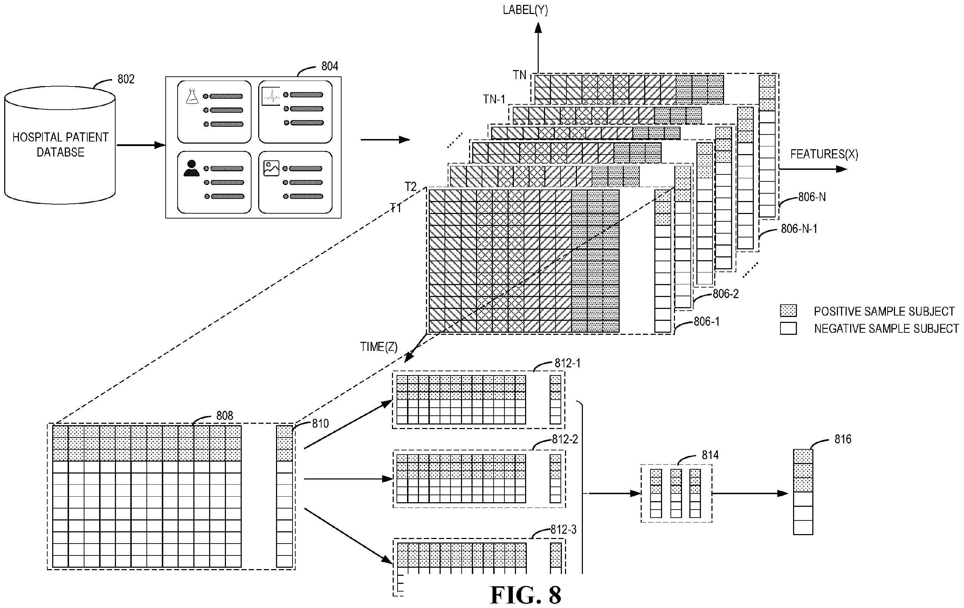

Reference is now made to Fig. 8, which illustrates the process for training a machine learning model in accordance with example embodiments of the present disclosure. In Fig. 8, a hospital patient database 802 stores data related to a large amounts of sample patients for training machine learning models. A plurality of sample clinical parameters 804 of a plurality of sample patients may be obtained from the hospital patient database 802. The plurality sample patients include positive cases and negative cases. The plurality of sample clinical parameters 804 include vital sign data, lab measurement data, waveform feature data, and image data. The embodiments in Fig. 8 are provided for illustration, rather than limitation. In some embodiments, the plurality of sample clinical parameters 804 includes at least two of vital sign data, lab measurement data, waveform feature data, and image data.

The plurality of sample clinical parameters for a plurality of sample subjects may vary over time. Therefore, the plurality of sample clinical parameters at different time points may be obtained. As shown in Fig. 8, there are data segments 806-1, 806-2, ..., 806-N-1, and 806-N corresponding to time points T1, T2, ..., TN-1, and TN respectively. Each data segment includes the plurality of sample clinical parameters for a plurality of sample subjects. Each line of the data segment represents the data for a sample subject. The data segment 806-1 corresponding to the time point T1 includes a plurality of sample clinical parameters 808 for a plurality of sample subjects and real results 810 of the hemodynamic instability for the plurality of sample subjects. In some embodiments, the plurality of sample subjects includes positive sample subjects and 9 negative sample subjects. The same number of the positive samples and negative samples are combined to train the sub-models. Therefore, the sample clinical parameters of the 3 positive sample subjects are combined with sample clinical parameters of 3 negative samples to form 3 sub-data segments 812-1, 812-2, and 812-3 to train 3 sub-models to obtain prediction results 814. The prediction results 814 includes 3 results, each of which is obtained from one of the 3 sub-models. The prediction results 814 are compared with the real results of hemodynamic instability for sample subjects to adjust the parameters of the 3 sub-models. The prediction results 814 are used to generate the combined prediction probability 816.

As shown in Fig. 8, the N data segments are used to train N machine learning models. The N machine learning models form a detection model to detect the hemodynamic instability. For each of machine learning model, the combined prediction probability is compared with a predetermined probability threshold. Therefore, the predetermined probability threshold is needed to improve performance. As mentioned before, the default value of the predetermined probability threshold is 0.5 in one example. In some embodiments, the default value can be updated based on the medical environment data in its location and the clinician's experience.

The detection model is a dynamic time varying model used to monitor patients and the predetermined ratio related to model performance may be determined from accuracy rate, precision rate, recall rate, a specificity rate, an F 1 score, false positive rate, true positive rate (TPR), and true negative rate (TNR). The following formulas are used to compute the above parameters related to model performance. More particularly, the true positives are determined by the following formula (1).

$$True\ positive\ (TP) = \sum_{i=1}^{n}(percentile\ \hat{P}_t^i | P_{g=1}^i), i\epsilon n, t\epsilon[-24,-1]\ (1)$$

Wherein $\hat{P}$ represents predicted values, $n$ represents patients counts, $t$ represents time in hours, g represents ground truth, $P_{g=1}^i$ represents that the ground truth of a patient i is 1, $P_{g=0}^i$ represents that ground truth of a patient i is 0, and *percentile* represents the predetermined ratio discussed as above. The sum of the true positives and false positives is determined by the following formula (2).

$$True\ positive\ (TP) + False\ positive\ (FP) = \sum_{i=1}^{n}\left(percentile\ \hat{P}_t^i\right), i\epsilon n, t\epsilon[-24,-1]\ (2)$$

The true negatives are determined by the following formula (3).

$$True\ negative\ (TN) = \sum_{i=1}^{n}((1-percentile)\ \hat{P}_t^i | P_{g=0}^i), i \in n, t \in [-1,-24]\ (3)$$

The sum of true positives and false negatives is determined from the following formula (4).

$$True\ positive\ (TP) + False\ negative\ (FN) = \sum_{i=1}^{n} P_{g=1}^i\quad (4)$$

The accuracy rate is determined based on the following formula (5).

$$ACCURATE = \frac{TP+TN}{TP+FP+TN+FN};$$

$$ACCURATE = \frac{\sum_{i=1}^{n}(percentile\ \hat{P}_t^i | P_{g=1}^i) + \sum_{i=1}^{n}((1-percentile)\ \hat{P}_t^i | P_{g=0}^i)}{n}\quad (5)$$

The precision rate is determined based on the following formula (6).

$$Precision = \frac{TP}{TP+FP};$$

$$Precision = \frac{\sum_{i=1}^{n}(percentile\ \hat{P}_t^i|P_{g=1}^i)}{\sum_{i=1}^{n}(percentile\ \hat{P}_t^i)} \quad (6)$$

The recall rate is determined based on the following formula (7).

$$Recall = TPR = \frac{TP}{TP+FN};$$

$$Recall = TPR = \frac{\sum_{i=1}^{n}(percentile\ \hat{P}_t^i|P_{g=1}^i),}{\sum_{i=1}^{n}P_{g=1}^i} \quad (7)$$

The specificity rate is determined based on the following formula (8).

$$Specificity = TNR = \frac{TN}{TN+FP}$$

$$Specificity = TNR =$$

$$\frac{\sum_{i=1}^{n}((1-percentile)\ \hat{P}_t^i|P_{g=0}^i)}{\sum_{i=1}^{n}((1-percentile)\ \hat{P}_t^i|P_{g=0}^i) +\sum_{i=1}^{n}(percentile\ \hat{P}_t^i)- \sum_{i=1}^{n}(percentile\ \hat{P}_t^i|P_{g=1}^i)} \quad (8)$$

The false positive rate is determined based on the following formula (9).

$$FPR = 1 - TNR(Specificity) = 1 -$$

$$\frac{\sum_{i=1}^{n}((1-percentile)\ \hat{P}_t^i|P_{g=0}^i)}{\sum_{i=1}^{n}((1-percentile)\ \hat{P}_t^i|P_{g=0}^i) +\sum_{i=1}^{n}(percentile\ \hat{P}_t^i)- \sum_{i=1}^{n}(percentile\ \hat{P}_t^i|P_{g=1}^i)} \quad (9)$$

The F1 score is determined based on the following formula (10).

$$F1 = \frac{2*(Precision*Recall)}{Precision*Recall};$$

$$F1 = \frac{2*\left(\frac{\sum_{i=1}^{n}(percentile\ \hat{P}_t^i|P_{g=1}^i)}{\sum_{i=1}^{n}(percentile\ \hat{P}_t^i)}\right)*\left(\frac{\sum_{i=1}^{n}(percentile\ \hat{P}_t^i|P_{g=1}^i),}{\sum_{i=1}^{n}P_{g=1}^i}\right)}{\left(\frac{\sum_{i=1}^{n}(percentile\ \hat{P}_t^i|P_{g=1}^i)}{\sum_{i=1}^{n}(percentile\ \hat{P}_t^i)}\right)*\left(\frac{\sum_{i=1}^{n}(percentile\ \hat{P}_t^i|P_{g=1}^i),}{\sum_{i=1}^{n}P_{g=1}^i}\right)} \quad (10)$$

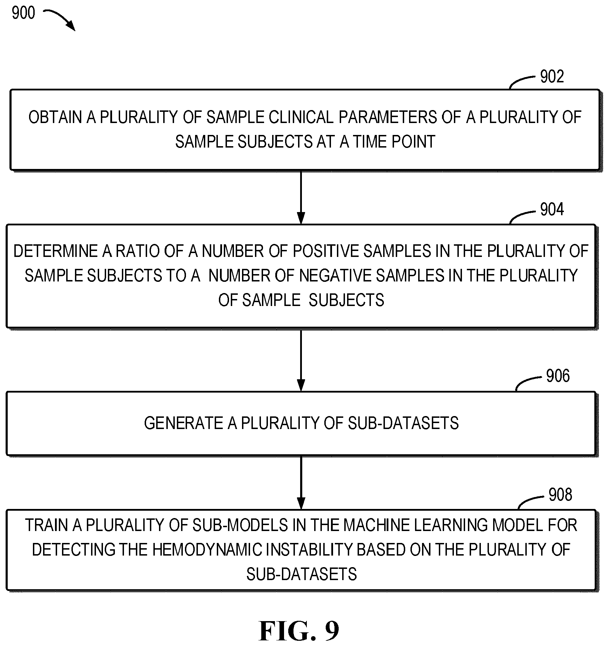

Reference is now made to Fig. 9, which illustrates an example method 900 for training a machine learning model for detecting hemodynamic instability in accordance with example embodiments of the present disclosure. The example process may be performed at the computing device 610 or any other suitable computing device.

At block 902, the computing device 610 obtains a plurality of sample clinical parameters of a plurality of sample subjects at a time point. The plurality of sample clinical parameters including at least two of vital sign data, lab measurement data, waveform feature data, and image data.

In some embodiments, when obtaining a plurality of sample clinical parameters of a plurality of sample subjects at a time point, the computing device 610 will check each of the plurality of sample clinical parameters. The computing device 610 may determine whether a sample clinical parameter of the plurality of sample clinical parameters is null. If the sample clinical parameter of the plurality of sample clinical parameters is not null, it will be used. If the sample clinical parameter of the plurality of sample clinical parameters is null, the computing device 610 determines a first filter time limit for sample clinical parameter. Then, the computing device 610 checks whether latest data for the sample clinical parameter is in the first filter time limit. If the latest data for the sample clinical parameter is in the first filter time limit, the sample clinical parameter is updated as the latest data. If there is no latest data for the sample clinical parameter in the first filter time limit, the sample clinical parameter will remain null. The filter time limits for the plurality of sample clinical parameters may be the same or different.

At block 904, the computing device 610 determines a ratio of a number of positive samples in the plurality of sample subjects to a number of negative samples in the plurality of sample subjects. The plurality of sample subjects may include positive samples and negative samples. However, the positive samples and negative samples for the hemodynamic instability are unbalanced. Therefore, the positive samples and negative samples need to be balanced based on the ratio.

At block 906, the computing device 610 generates a plurality of sub-datasets. Each sub-dataset includes sample clinical parameters corresponding to the positive samples and sample clinical parameters corresponding to one portion of the negative samples. The negative samples are divided into a number of portions based on the ratio, and the one portion of the negative samples for the each sub-dataset is different from each other. In order to obtain the sub-datasets used to train the sub-models of the machine learning model, the computing device 610 combines the sample clinical parameters of the positive samples with the sample clinical parameters of a plurality of different parts of the negative samples respectively to form a plurality of sub-datasets. Therefore, each of the plurality of sub-datasets includes data from the same positive samples and different negative samples.

At block 908, the computing device 610 trains a plurality of sub-models in the machine learning model for detecting the hemodynamic instability based on the plurality of sub-datasets. The computing device 610 uses the plurality of sub-datasets to train a plurality of sub-models. In some embodiments, one of the plurality of sub-datasets is used to train one of the plurality of sub-models. In some embodiments, two or more of the plurality of sub-datasets may be used to train one of the plurality of sub-models.

In some embodiments, when training a plurality of sub-models in the machine learning model, the computing device 610 obtains a prediction probability of a sub-model of the plurality of sub-models by inputting a sub-dataset of the plurality of sub-datasets into the sub-model. Then, the computing device 610 compares the prediction probability and a real result of hemodynamic instability, and adjusts model parameters of the sub-model based on the comparison.

In some embodiments, the machine learning model is one of a plurality of machine learning models, the plurality of machine learning models form a detection model for detecting the hemodynamic instability. The computing device 610 will obtain a further plurality of sample clinical parameters of the plurality of sample subjects at a further time point different from the time point. For example, the time point is 5 o'clock and the further time point is 6 o'clock. The computing device 610 trains a further machine learning model of the plurality of machine learning models using the further plurality of sample clinical parameters. In this way, the computing device will train a plurality of machine learning models using the data of the corresponding time points. Each of the plurality of machine learning models is associated with a time point.

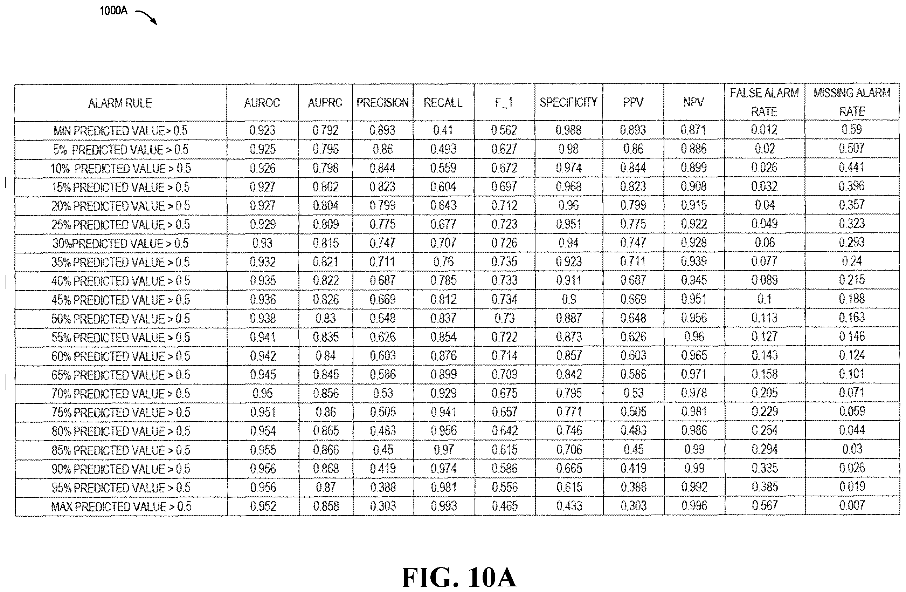

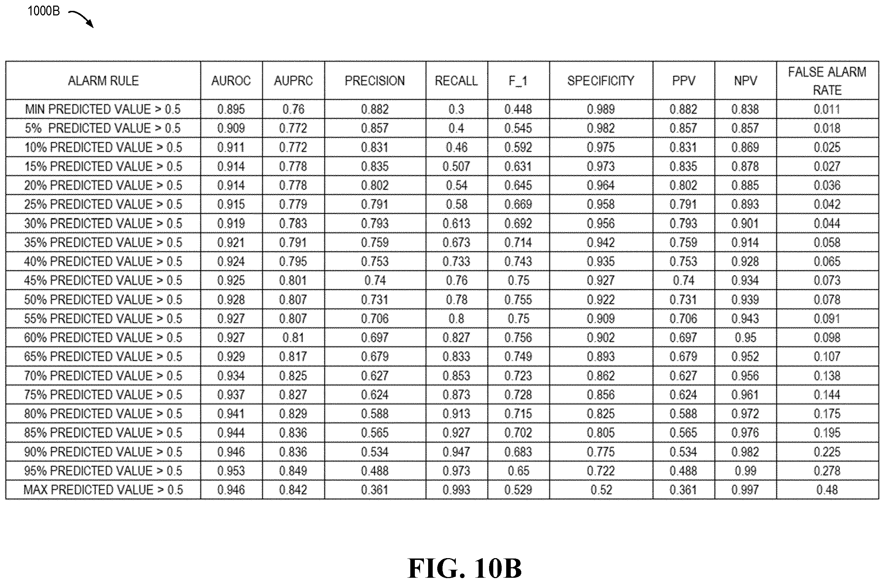

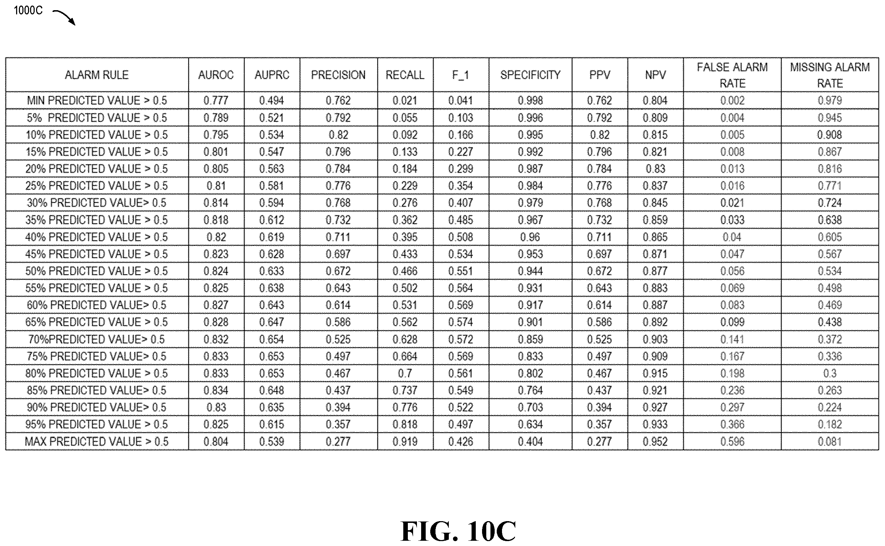

Reference is now made to Figs. 10A, 10B, and 10C, which show tables illustrating model performances of the detection model for detecting hemodynamic instability in accordance with example embodiments of the present disclosure. Fig. 10A shows the example table 1000A of the model performance for TPEVGH, which is internal testing results of the detection model for detecting the hemodynamic instability. Fig. 10B shows the example table 1000B of the model performance for TPEVGH 2022, which is external testing results of the detection model for detecting the hemodynamic instability. Fig. 10C shows the example table 1000C of the model performance for MIMIC IV, which is also external testing results of the detection model for detecting the hemodynamic instability. As shown in Figs. 10A, 10B, and 10C, the area under receiver operating characteristic (AUROC), the area under the Precision-Recall curve (AUPRC), the positive predictive value (PPV), the negative predictive value (NPV), the precision, the $F_1$, and the specificity are significantly improved. For the internal testing results and external testing results, the model demonstrates good ability for detect positive cases.

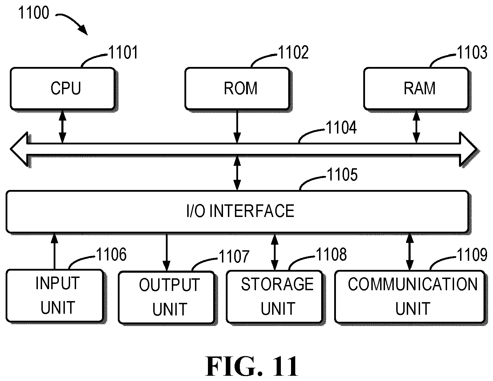

Reference is now made to Fig. 11 which illustrates a schematic block diagram of an example device 1100 for implementing embodiments of the present disclosure. The computing device 110 in Fig. 1 and the computing device 610 in Fig. 6 may be implemented by the device. As shown in Fig. 11, the device 1100 comprises a central process unit (CPU) 1101, which can execute various suitable actions and processing based on the computer program instructions stored in a read-only memory (ROM) 1102 or computer program instructions loaded in the random-access memory (RAM) 1103 from a storage unit 1108. The RAM 1103 can also store all kinds of programs and data required by the operation of the device 1100. CPU 1101, ROM 1102 and RAM 1103 are connected to each other via a bus 1104. The input/output (I/O) interface 1105 is also connected to the bus 1104.

A plurality of components in the device 1100 is connected to the I/O interface 1105, including: an input unit 1106, such as a keyboard, a mouse and the like; an output unit 1107, e.g., various kinds of display and loudspeakers etc.; a storage unit 1108, such as a disk and an optical disk etc.; and a communication unit 1109, such as a network card, a modem, a wireless transceiver and the like. The communication unit 1109 allows the device 1100 to exchange information/data with other devices via the computer network, such as Internet, and/or various telecommunication networks.

The above described procedure and processing, such as methods 500 and 900, can be executed by the processing unit 1101. For example, in some embodiments, the methods 500 and 900 can be implemented as a computer software program tangibly included in the machine-readable medium, e.g., storage unit 1108. In some embodiments, the computer program can be partially or fully loaded and/or mounted to the device 1100 via ROM 1102 and/or communication unit 1109. When the computer program is loaded to RAM 1103 and executed by the CPU 1101, one or more actions of the above describe methods 500 and 900 can be implemented.

The present disclosure can be a method, apparatus, system and/or computer program product. The computer program product can include a computer-readable storage medium, on which the computer-readable program instructions for executing various aspects of the present disclosure are loaded.

The computer-readable storage medium can be a tangible apparatus that maintains and stores instructions utilized by the instruction executing apparatuses. The computer-readable storage medium can be, but not limited to, such as an electrical storage device, a magnetic storage device, an optical storage device, an electromagnetic storage device, a semiconductor storage device or any appropriate combinations of the above. More concrete examples of the computer-readable storage medium (non-exhaustive list) include: a portable computer disk, a hard disk, a random-access memory (RAM), a read-only memory (ROM), an erasable programmable read-only memory (EPROM or flash), a static random-access memory (SRAM), a portable compact disk read-only memory (CD-ROM), a digital versatile disk (DVD), a memory stick, a floppy disk, mechanical coding devices, a punched card stored with instructions thereon, or a projection in a slot, and any appropriate combinations of the above. The computer-readable storage medium utilized here is not interpreted as transient signals per se, such as radio waves or freely propagated electromagnetic waves, electromagnetic waves propagated via waveguide or other transmission media (such as optical pulses via fiber-optic cables), or electric signals propagated via electric wires.

The described computer-readable program instruction can be downloaded from the computer-readable storage medium to each computing/processing device, or to an external computer or external storage via Internet, local area network, wide area network and/or wireless network. The network can comprise copper-transmitted cable, optical fiber transmission, wireless transmission, router, firewall, switch, network gate computer and/or edge server. The network adapter card or network interface in each computing/processing device receives computer-readable program instructions from the network and forwards the computer-readable program instructions for storage in the computer-readable storage medium of each computing/processing device.

The computer program instructions for executing operations of the present disclosure can be assembly instructions, instructions of instruction set architecture (ISA), machine instructions, machine-related instructions, micro codes, firmware instructions, state setting data, or source codes or target codes written in any combinations of one or more programming languages, wherein the programming languages comprise object-oriented programming languages, e.g., Smalltalk, C++ and so on, and traditional procedural programming languages, such as "C" language or similar programming languages. The computer-readable program instructions can be implemented fully on the user computer, partially on the user computer, as an independent software package, partially on the user computer and partially on the remote computer, or completely on the remote computer or server. In the case where remote computer is involved, the remote computer can be connected to the user computer via any type of networks, including local area network (LAN) and wide area network (WAN), or to the external computer (e.g., connected via Internet using the Internet service provider). In some embodiments, state information of the computer-readable program instructions is used to customize an electronic circuit, e.g., programmable logic circuit, field programmable gate array (FPGA) or programmable logic array (PLA). The electronic circuit can execute computer-readable program instructions to implement various aspects of the present disclosure.

Various aspects of the present disclosure are described here with reference to flow chart and/or block diagram of method, apparatus (system) and computer program products according to embodiments of the present disclosure. It should be understood that each block of the flow chart and/or block diagram and the combination of various blocks in the flow chart and/or block diagram can be implemented by computer-readable program instructions.

The computer-readable program instructions can be provided to the processing unit of general-purpose computer, dedicated computer or other programmable data processing apparatuses to manufacture a machine, such that the instructions that, when executed by the processing unit of the computer or other programmable data processing apparatuses, generate an apparatus for implementing functions/actions stipulated in one or more blocks in the flow chart and/or block diagram. The computer-readable program instructions can also be stored in the computer-readable storage medium and cause the computer, programmable data processing apparatus and/or other devices to work in a particular manner, such that the computer-readable medium stored with instructions comprises an article of manufacture, including instructions for implementing various aspects of the functions/actions stipulated in one or more blocks of the flow chart and/or block diagram.

The computer-readable program instructions can also be loaded into computer, other programmable data processing apparatuses or other devices, so as to execute a series of operation steps on the computer, other programmable data processing apparatuses or other devices to generate a computer-implemented procedure. Therefore, the instructions executed on the computer, other programmable data processing apparatuses or other devices implement functions/actions stipulated in one or more blocks of the flow chart and/or block diagram.

The flow chart and block diagram in the drawings illustrate system architecture, functions and operations that may be implemented by system, method and computer program product according to multiple implementations of the present disclosure. In this regard, each block in the flow chart or block diagram can represent a module, a part of program segment or code, wherein the module and the part of program segment or code include one or more executable instructions for performing stipulated logic functions. In some alternative implementations, it should be noted that the functions indicated in the block can also take place in an order different from the one indicated in the drawings. For example, two successive blocks can be in fact executed in parallel or sometimes in a reverse order dependent on the involved functions. It should also be noted that each block in the block diagram and/or flow chart and combinations of the blocks in the block diagram and/or flow chart can be implemented by a hardware-based system exclusive for executing stipulated functions or actions, or by a combination of dedicated hardware and computer instructions.

Various embodiments of the present disclosure have been described above and the above description is only exemplary rather than exhaustive and is not limited to the embodiments of the present disclosure. Many modifications and alterations, without deviating from the scope and spirit of the explained various embodiments, are obvious for those skilled in the art. The selection of terms in the text aims to best explain principles and actual applications of each embodiment and technical improvements made in the market by each embodiment, or enable those ordinary skilled in the art to understand embodiments of the present disclosure.

## Claims

1.   A method of detecting hemodynamic instability, comprising:

obtaining a plurality of clinical parameters of a subject at a time point, the plurality of clinical parameters including at least two of vital sign data, lab measurement data, waveform feature data, and image data;
determining, by a machine learning model, a plurality of prediction probabilities based on the plurality of clinical parameters, each of the plurality of prediction probabilities being determined by a corresponding sub-model of a plurality of sub-models in the machine learning model;
determining a combined prediction probability for the time point based on the plurality of prediction probabilities; and
detecting the hemodynamic instability based on the combined prediction probability.

2.   The method according to claim 1, wherein

the vital sign data includes at least one of a hearth rate, a temperature, a respiration rate, and a blood pressure;
the lab measurement data includes at least one of creatinine, blood sugar, glutamic acid, lactic acid, alanine transaminase, and aspartate transaminase;
the waveform feature data includes at least one of a peak-to-trough slope of a heart rate waveform, spacing between peaks of the heart rate waveform, a peak-to-trough slope of a ventilator waveform, and spacing between peaks of the ventilator waveform; and/or
the image data includes at least one of an X-ray image, an ultrasound image, and a computed tomography image.

3.   The method according to claim 1, wherein obtaining a plurality of clinical parameters of a subject at a time point comprises:

determining whether a clinical parameter of the plurality of clinical parameters is null;
in response to determining that the clinical parameter of the plurality of clinical parameters is null, determining whether latest data for the clinical parameter exists in a first filter time limit; and
in response to determining that the latest data for the clinical parameter exists in the first filter time limit, updating the clinical parameter based on the latest data.

4.   The method according to claim 3, wherein obtaining a plurality of clinical parameters of a subject at a time point further comprises:

determining whether a further clinical parameter of the plurality of clinical parameters is null;
in response to determining that the further clinical parameter of the plurality of clinical parameters is null, determining whether further latest data for the further clinical parameter exists in a second filter time limit, the second filter time limit being different from the first filter time limit; and
in response to determining that the further latest data for the further clinical parameter exists in the second filter time limit, updating the further clinical parameter based on the further latest data.

5. The method according to claim 1, wherein the machine learning model is one of a plurality of machine learning models, the plurality of machine learning models form a detection model for detecting the hemodynamic instability, and each of the plurality of machine learning models is used to determine a combined prediction probability at a corresponding time point of a plurality of time points.

6. The method according to claim 5, wherein determining a combined prediction probability for the time point based on the plurality of prediction probabilities comprises:

for each corresponding time point of the plurality of time points, determining a combined prediction probability by using a machine learning model respectively corresponding to the each corresponding time point based on the plurality of clinical parameters of the subject obtained at the each corresponding time point to obtain a plurality of combined prediction probabilities;
wherein detecting the hemodynamic instability based on the combined prediction probability comprises:

selecting a predetermined number of combined prediction probabilities from the plurality of combined prediction probabilities, each selected combined prediction probabilities being greater than the remaining combined prediction probabilities of the plurality of combined prediction probabilities;
determining whether each of the predetermined number of combined prediction probabilities is greater than a predetermined probability threshold; and
in response to determining that each of the predetermined number of combined prediction probabilities is greater than the predetermined probability threshold, determining that the hemodynamic instability occurs.

7. The method according to claim 6, further comprising:

determining a predetermined ratio related to a model performance of the detection model based on at least one of: an accuracy rate, a precision rate, a recall rate, a specificity rate, an F1 score, and a false positive rate of the detection model; and
determining the predetermined number based on the predetermined ratio and the number of the plurality of machine learning models.

8. The method according to claim 5, wherein detecting the hemodynamic instability based on the combined prediction probability comprises:

obtaining a plurality of clinical parameters of the subject for each of at least two further time points different from the time point;
for the each of at least two further time points, determining a combined prediction probability, by using a machine learning model respectively corresponding to the each of at least two further time points based on the plurality of clinical parameters obtained at the each of at least further time points; and
detecting the hemodynamic instability based on the combined prediction probability for the time point and the combined prediction probabilities respectively for the at least two further time points.

9. The method according to claim 1, wherein determining a combined prediction probability for the time point based on the plurality of prediction probabilities comprises:
determining an average probability of the plurality of prediction probabilities as the combined prediction probability.

10. A method of training a machine learning model for detecting hemodynamic instability, comprising:

obtaining a plurality of sample clinical parameters of a plurality of sample subjects at a time point, the plurality of sample clinical parameters including at least two of vital sign data, lab measurement data, waveform feature data, and image data;
determining a ratio of a number of positive samples in the plurality of sample subjects to a number of negative samples in the plurality of sample subjects;
generating a plurality of sub-datasets, each sub-dataset including sample clinical parameters corresponding to the positive samples and sample clinical parameters corresponding to one portion of the negative samples, wherein the negative samples are divided into a number of portions based on the ratio, and the one portion of the negative samples for the each sub-dataset is different from each other; and
training a plurality of sub-models in the machine learning model for detecting the hemodynamic instability based on the plurality of sub-datasets.

11. The method according to claim 10, wherein the machine learning model is one of a plurality of machine learning models, the plurality of machine learning models form a detection model for detecting the hemodynamic instability, and the method further comprises:

obtaining a further plurality of sample clinical parameters of the plurality of sample subjects at a further time point different from the time point; and
training a further machine learning model of the plurality of machine learning models based on the further plurality of sample clinical parameters.

12. The method according to claim 10, wherein obtaining a plurality of sample clinical parameters of a plurality of sample subjects at a time point comprises:

determining whether a sample clinical parameter of the plurality of sample clinical parameters is null;
in response to determining that the sample clinical parameter of the plurality of sample clinical parameters is null, determining whether latest data for the sample clinical parameter exists in a first filter time limit; and
in response to determining that the latest data for the sample clinical parameter exists in the first filter time limit, updating the sample clinical parameter based on the latest data.

13. The method according to claim 12, wherein training a plurality of sub-models in the machine learning model for detecting the hemodynamic instability based on the plurality of sub-datasets comprises:

obtaining a prediction probability of a sub-model of the plurality of sub-models by inputting a sub-dataset of the plurality of sub-datasets into the sub-model; and
adjusting model parameters of the sub-model based on the prediction probability and a real result of hemodynamic instability.

14. An electronic device, comprising:

at least one processor; and
at least one memory having a plurality of instructions stored thereon, when executed by the at least one processor, cause the device to perform the method according to any of claims 1-9 or claims 10-13.

15. A computer readable medium having computer instructions stored thereon, the computer instructions, when executed a processor, causing the processor to perform the method according to any of claims 1-9 or claims 10-13.

100

| 102 | 104 | 106 | 108 |
|---|---|---|---|
| VITAL SIGN DATA | LAB MEASUREMENT DATA | WAVEFORM FEATURE DATA | IMAGE DATA |

110

COMPUTING DEVICE

112

MACHINE LEARNING MODEL

| 114-1 | 114-2 | | 114-M |
|---|---|---|---|
| SUB-MODEL | SUB-MODEL | å å | SUB-MODEL |

116

HEMODYNAMIC INSTABILITY

**FIG. 1**

**FIG. 2**

FIG. 3

400

| PATIENT | AGE | HR | CVP | CREATININE | TEMPERATURE | å å | Y |
|---------|-----|-----|-----|------------|-------------|-----|---|
| 001 | 66 | 90 | FIND THE LASTEST MEASURED VALUE WITHIN 26 HOURS | FIND THE LASTEST MEASURED VALUE WITHIN 26 HOURS | 37°C | • FIND THE LASTEST MEASURED VALUE WITHIN 26 HOURS (LAB INDICATORS)<br>• FIND THE LASTEST MEASURED VALUE WITHIN 2 HOURS (VITAL INDICATORS) | HEMO INSTABILITY |

# FIG. 4

500

502

OBTAIN A PLURALITY OF CLINICAL PARAMETERS OF A SUBJECT AT A TIME POINT

504

DETERMINE, BY A MACHINE LEARNING MODEL, A PLURALITY OF PREDICTION PROBABILITIES BASED ON THE PLURALITY OF CLINICAL PARAMETERS

506

DETERMINE A COMBINED PREDICTION PROBABILITY FOR THE TIME POINT BASED ON THE PLURALITY OF PREDICTION PROBABILITIES

508

DETECT THE HEMODYNAMIC INSTABILITY BASED ON THE COMBINED PREDICTION PROBABILITY

## FIG. 5

600

| 602 | 604 | 606 | 608 |
|---|---|---|---|
| VITAL SIGN DATA | LAB MEASUREMENT DATA | WAVEFORM FEATURE DATA | IMAGE DATA |

610

**COMPUTING DEVICE**

612

**DATASET**

| 614-1 | 614-2 | 614-T |
|---|---|---|
| SUB-DATASET | SUB-DATASET | SUB-DATASET |

616

**MACHINE LEARNING MODEL**

| 618-1 | 618-2 | 618-M |
|---|---|---|
| SUB-MODEL | SUB-MODEL | SUB-MODEL |

620

**HEMODYNAMIC INSTABILITY**

**FIG. 6**

**FIG. 7**

FIG. 8

EP 4 528 750 A1

900

902

OBTAIN A PLURALITY OF SAMPLE CLINICAL PARAMETERS OF A PLURALITY OF SAMPLE SUBJECTS AT A TIME POINT

904

DETERMINE A RATIO OF A NUMBER OF POSITIVE SAMPLES IN THE PLURALITY OF SAMPLE SUBJECTS TO A NUMBER OF NEGATIVE SAMPLES IN THE PLURALITY OF SAMPLE SUBJECTS

906

GENERATE A PLURALITY OF SUB-DATASETS

908

TRAIN A PLURALITY OF SUB-MODELS IN THE MACHINE LEARNING MODEL FOR DETECTING THE HEMODYNAMIC INSTABILITY BASED ON THE PLURALITY OF SUB-DATASETS

# FIG. 9

1000A

| ALARM RULE | AUROC | AUPRC | PRECISION | RECALL | F_1 | SPECIFICITY | PPV | NPV | FALSE ALARM RATE | MISSING ALARM RATE |
|---|---|---|---|---|---|---|---|---|---|---|
| MIN PREDICTED VALUE> 0.5 | 0.923 | 0.792 | 0.893 | 0.41 | 0.562 | 0.988 | 0.893 | 0.871 | 0.012 | 0.59 |
| 5% PREDICTED VALUE > 0.5 | 0.925 | 0.796 | 0.86 | 0.493 | 0.627 | 0.98 | 0.86 | 0.886 | 0.02 | 0.507 |
| 10% PREDICTED VALUE > 0.5 | 0.926 | 0.798 | 0.844 | 0.559 | 0.672 | 0.974 | 0.844 | 0.899 | 0.026 | 0.441 |
| 15% PREDICTED VALUE > 0.5 | 0.927 | 0.802 | 0.823 | 0.604 | 0.697 | 0.968 | 0.823 | 0.908 | 0.032 | 0.396 |
| 20% PREDICTED VALUE > 0.5 | 0.927 | 0.804 | 0.799 | 0.643 | 0.712 | 0.96 | 0.799 | 0.915 | 0.04 | 0.357 |
| 25% PREDICTED VALUE > 0.5 | 0.929 | 0.809 | 0.775 | 0.677 | 0.723 | 0.951 | 0.775 | 0.922 | 0.049 | 0.323 |
| 30%PREDICTED VALUE > 0.5 | 0.93 | 0.815 | 0.747 | 0.707 | 0.726 | 0.94 | 0.747 | 0.928 | 0.06 | 0.293 |
| 35% PREDICTED VALUE > 0.5 | 0.932 | 0.821 | 0.711 | 0.76 | 0.735 | 0.923 | 0.711 | 0.939 | 0.077 | 0.24 |
| 40% PREDICTED VALUE > 0.5 | 0.935 | 0.822 | 0.687 | 0.785 | 0.733 | 0.911 | 0.687 | 0.945 | 0.089 | 0.215 |
| 45% PREDICTED VALUE > 0.5 | 0.936 | 0.826 | 0.669 | 0.812 | 0.734 | 0.9 | 0.669 | 0.951 | 0.1 | 0.188 |
| 50% PREDICTED VALUE > 0.5 | 0.938 | 0.83 | 0.648 | 0.837 | 0.73 | 0.887 | 0.648 | 0.956 | 0.113 | 0.163 |
| 55% PREDICTED VALUE > 0.5 | 0.941 | 0.835 | 0.626 | 0.854 | 0.722 | 0.873 | 0.626 | 0.96 | 0.127 | 0.146 |
| 60% PREDICTED VALUE > 0.5 | 0.942 | 0.84 | 0.603 | 0.876 | 0.714 | 0.857 | 0.603 | 0.965 | 0.143 | 0.124 |
| 65% PREDICTED VALUE > 0.5 | 0.945 | 0.845 | 0.586 | 0.899 | 0.709 | 0.842 | 0.586 | 0.971 | 0.158 | 0.101 |
| 70% PREDICTED VALUE > 0.5 | 0.95 | 0.856 | 0.53 | 0.929 | 0.675 | 0.795 | 0.53 | 0.978 | 0.205 | 0.071 |
| 75% PREDICTED VALUE > 0.5 | 0.951 | 0.86 | 0.505 | 0.941 | 0.657 | 0.771 | 0.505 | 0.981 | 0.229 | 0.059 |
| 80% PREDICTED VALUE > 0.5 | 0.954 | 0.865 | 0.483 | 0.956 | 0.642 | 0.746 | 0.483 | 0.986 | 0.254 | 0.044 |
| 85% PREDICTED VALUE > 0.5 | 0.955 | 0.866 | 0.45 | 0.97 | 0.615 | 0.706 | 0.45 | 0.99 | 0.294 | 0.03 |
| 90% PREDICTED VALUE > 0.5 | 0.956 | 0.868 | 0.419 | 0.974 | 0.586 | 0.665 | 0.419 | 0.99 | 0.335 | 0.026 |
| 95% PREDICTED VALUE > 0.5 | 0.956 | 0.87 | 0.388 | 0.981 | 0.556 | 0.615 | 0.388 | 0.992 | 0.385 | 0.019 |
| MAX PREDICTED VALUE > 0.5 | 0.952 | 0.858 | 0.303 | 0.993 | 0.465 | 0.433 | 0.303 | 0.996 | 0.567 | 0.007 |

**FIG. 10A**

1000B

| ALARM RULE | AUROC | AUPRC | PRECISION | RECALL | F_1 | SPECIFICITY | PPV | NPV | FALSE ALARM RATE |
|---|---|---|---|---|---|---|---|---|---|
| MIN PREDICTED VALUE > 0.5 | 0.895 | 0.76 | 0.882 | 0.3 | 0.448 | 0.989 | 0.882 | 0.838 | 0.011 |
| 5% PREDICTED VALUE > 0.5 | 0.909 | 0.772 | 0.857 | 0.4 | 0.545 | 0.982 | 0.857 | 0.857 | 0.018 |
| 10% PREDICTED VALUE > 0.5 | 0.911 | 0.772 | 0.831 | 0.46 | 0.592 | 0.975 | 0.831 | 0.869 | 0.025 |
| 15% PREDICTED VALUE > 0.5 | 0.914 | 0.778 | 0.835 | 0.507 | 0.631 | 0.973 | 0.835 | 0.878 | 0.027 |
| 20% PREDICTED VALUE > 0.5 | 0.914 | 0.778 | 0.802 | 0.54 | 0.645 | 0.964 | 0.802 | 0.885 | 0.036 |
| 25% PREDICTED VALUE > 0.5 | 0.915 | 0.779 | 0.791 | 0.58 | 0.669 | 0.958 | 0.791 | 0.893 | 0.042 |
| 30% PREDICTED VALUE > 0.5 | 0.919 | 0.783 | 0.793 | 0.613 | 0.692 | 0.956 | 0.793 | 0.901 | 0.044 |
| 35% PREDICTED VALUE > 0.5 | 0.921 | 0.791 | 0.759 | 0.673 | 0.714 | 0.942 | 0.759 | 0.914 | 0.058 |
| 40% PREDICTED VALUE > 0.5 | 0.924 | 0.795 | 0.753 | 0.733 | 0.743 | 0.935 | 0.753 | 0.928 | 0.065 |
| 45% PREDICTED VALUE > 0.5 | 0.925 | 0.801 | 0.74 | 0.76 | 0.75 | 0.927 | 0.74 | 0.934 | 0.073 |
| 50% PREDICTED VALUE > 0.5 | 0.928 | 0.807 | 0.731 | 0.78 | 0.755 | 0.922 | 0.731 | 0.939 | 0.078 |
| 55% PREDICTED VALUE > 0.5 | 0.927 | 0.807 | 0.706 | 0.8 | 0.75 | 0.909 | 0.706 | 0.943 | 0.091 |
| 60% PREDICTED VALUE > 0.5 | 0.927 | 0.81 | 0.697 | 0.827 | 0.756 | 0.902 | 0.697 | 0.95 | 0.098 |
| 65% PREDICTED VALUE > 0.5 | 0.929 | 0.817 | 0.679 | 0.833 | 0.749 | 0.893 | 0.679 | 0.952 | 0.107 |
| 70% PREDICTED VALUE > 0.5 | 0.934 | 0.825 | 0.627 | 0.853 | 0.723 | 0.862 | 0.627 | 0.956 | 0.138 |
| 75% PREDICTED VALUE > 0.5 | 0.937 | 0.827 | 0.624 | 0.873 | 0.728 | 0.856 | 0.624 | 0.961 | 0.144 |
| 80% PREDICTED VALUE > 0.5 | 0.941 | 0.829 | 0.588 | 0.913 | 0.715 | 0.825 | 0.588 | 0.972 | 0.175 |
| 85% PREDICTED VALUE > 0.5 | 0.944 | 0.836 | 0.565 | 0.927 | 0.702 | 0.805 | 0.565 | 0.976 | 0.195 |
| 90% PREDICTED VALUE > 0.5 | 0.946 | 0.836 | 0.534 | 0.947 | 0.683 | 0.775 | 0.534 | 0.982 | 0.225 |
| 95% PREDICTED VALUE > 0.5 | 0.953 | 0.849 | 0.488 | 0.973 | 0.65 | 0.722 | 0.488 | 0.99 | 0.278 |
| MAX PREDICTED VALUE > 0.5 | 0.946 | 0.842 | 0.361 | 0.993 | 0.529 | 0.52 | 0.361 | 0.997 | 0.48 |

**FIG. 10B**

| ALARM RULE | AUROC | AUPRC | PRECISION | RECALL | F_1 | SPECIFICITY | PPV | NPV | FALSE ALARM RATE | MISSING ALARM RATE |
|---|---|---|---|---|---|---|---|---|---|---|
| MIN PREDICTED VALUE > 0.5 | 0.777 | 0.494 | 0.762 | 0.021 | 0.041 | 0.998 | 0.762 | 0.804 | 0.002 | 0.979 |
| 5% PREDICTED VALUE > 0.5 | 0.789 | 0.521 | 0.792 | 0.055 | 0.103 | 0.996 | 0.792 | 0.809 | 0.004 | 0.945 |
| 10% PREDICTED VALUE > 0.5 | 0.795 | 0.534 | 0.82 | 0.092 | 0.166 | 0.995 | 0.82 | 0.815 | 0.005 | 0.908 |
| 15% PREDICTED VALUE > 0.5 | 0.801 | 0.547 | 0.796 | 0.133 | 0.227 | 0.992 | 0.796 | 0.821 | 0.008 | 0.867 |
| 20% PREDICTED VALUE > 0.5 | 0.805 | 0.563 | 0.784 | 0.184 | 0.299 | 0.987 | 0.784 | 0.83 | 0.013 | 0.816 |
| 25% PREDICTED VALUE > 0.5 | 0.81 | 0.581 | 0.776 | 0.229 | 0.354 | 0.984 | 0.776 | 0.837 | 0.016 | 0.771 |
| 30% PREDICTED VALUE> 0.5 | 0.814 | 0.594 | 0.768 | 0.276 | 0.407 | 0.979 | 0.768 | 0.845 | 0.021 | 0.724 |
| 35% PREDICTED VALUE > 0.5 | 0.818 | 0.612 | 0.732 | 0.362 | 0.485 | 0.967 | 0.732 | 0.859 | 0.033 | 0.638 |
| 40% PREDICTED VALUE > 0.5 | 0.82 | 0.619 | 0.711 | 0.395 | 0.508 | 0.96 | 0.711 | 0.865 | 0.04 | 0.605 |
| 45% PREDICTED VALUE > 0.5 | 0.823 | 0.628 | 0.697 | 0.433 | 0.534 | 0.953 | 0.697 | 0.871 | 0.047 | 0.567 |
| 50% PREDICTED VALUE > 0.5 | 0.824 | 0.633 | 0.672 | 0.466 | 0.551 | 0.944 | 0.672 | 0.877 | 0.056 | 0.534 |
| 55% PREDICTED VALUE > 0.5 | 0.825 | 0.638 | 0.643 | 0.502 | 0.564 | 0.931 | 0.643 | 0.883 | 0.069 | 0.498 |
| 60% PREDICTED VALUE> 0.5 | 0.827 | 0.643 | 0.614 | 0.531 | 0.569 | 0.917 | 0.614 | 0.887 | 0.083 | 0.469 |
| 65% PREDICTED VALUE > 0.5 | 0.828 | 0.647 | 0.586 | 0.562 | 0.574 | 0.901 | 0.586 | 0.892 | 0.099 | 0.438 |
| 70%PREDICTED VALUE> 0.5 | 0.832 | 0.654 | 0.525 | 0.628 | 0.572 | 0.859 | 0.525 | 0.903 | 0.141 | 0.372 |
| 75% PREDICTED VALUE> 0.5 | 0.833 | 0.653 | 0.497 | 0.664 | 0.569 | 0.833 | 0.497 | 0.909 | 0.167 | 0.336 |
| 80% PREDICTED VALUE > 0.5 | 0.833 | 0.653 | 0.467 | 0.7 | 0.561 | 0.802 | 0.467 | 0.915 | 0.198 | 0.3 |
| 85% PREDICTED VALUE> 0.5 | 0.834 | 0.648 | 0.437 | 0.737 | 0.549 | 0.764 | 0.437 | 0.921 | 0.236 | 0.263 |
| 90% PREDICTED VALUE> 0.5 | 0.83 | 0.635 | 0.394 | 0.776 | 0.522 | 0.703 | 0.394 | 0.927 | 0.297 | 0.224 |
| 95% PREDICTED VALUE > 0.5 | 0.825 | 0.615 | 0.357 | 0.818 | 0.497 | 0.634 | 0.357 | 0.933 | 0.366 | 0.182 |
| MAX PREDICTED VALUE > 0.5 | 0.804 | 0.539 | 0.277 | 0.919 | 0.426 | 0.404 | 0.277 | 0.952 | 0.596 | 0.081 |

**FIG. 10C**

**FIG. 11**

<table>
<tr><td>Europäisches Patentamt<br>European Patent Office<br>Office européen des brevets</td><td>**EUROPEAN SEARCH REPORT**</td><td>Application Number<br><br>**EP 23 21 3617**</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CRISTHIAN POTES: "A clinical prediction model to identify patients at high risk of hemodynamic instability in the pediatric intensive care unit",<br>CRITICAL CARE,<br>vol. 21, no. 1,<br>20 November 2017 (2017-11-20),<br>XP093153964,<br>GB<br>ISSN: 1364-8535, DOI: 10.1186/s13054-017-1874-z<br>Retrieved from the Internet:<br>URL:https://ccforum.biomedcentral.com/counter/pdf/10.1186/s13054-017-1874-z.pdf> | 1-9,14, 15 | INV.<br>G16H50/20<br>G16H50/30<br>G16H50/70 |
| A | * abstract; p.2, col.1, par.2; p.3, col.1, par.1; p.3, col.2, par.2; p.3, col.2, par.3; p.4, table 1; p.6, col.1, par.1 * | 10-13 | |
| A | J LEE: "A hypotensive episode predictor for intensive care based on heart rate and blood pressure time series",<br>COMPUTERS IN CARDIOLOGY, 2010,<br>22 March 2011 (2011-03-22), pages 81-84,<br>XP093153980,<br>ISBN: 978-1-4244-7318-2<br>Retrieved from the Internet:<br>URL:https://www.ncbi.nlm.nih.gov/pmc/articles/PMC3162312/pdf/nihms299859.pdf><br>* abstract; p.2, par.2; p.3, par.2; p.3, par.6 * | 1-15 | TECHNICAL FIELDS SEARCHED (IPC)<br><br>G16H |
| A | WO 2021/180500 A1 (KONINKLIJKE PHILIPS NV [NL]) 16 September 2021 (2021-09-16)<br>* the whole document * | 1-15 | |

The present search report has been drawn up for all claims

| Place of search | Date of completion of the search | Examiner |
|---|---|---|
| Munich | 23 April 2024 | Sundqvist, Benjamin |

## ANNEX TO THE EUROPEAN SEARCH REPORT
## ON EUROPEAN PATENT APPLICATION NO.

**EP 23 21 3617**

This annex lists the patent family members relating to the patent documents cited in the above-mentioned European search report.
The members are as contained in the European Patent Office EDP file on
The European Patent Office is in no way liable for these particulars which are merely given for the purpose of information.

**23-04-2024**

| Patent document<br>cited in search report | Publication<br>date | Patent family<br>member(s) | | Publication<br>date |
|---|---|---|---|---|
| WO 2021180500 A1 | 16-09-2021 | CN | 115279260 A | 01-11-2022 |
| | | EP | 4117514 A1 | 18-01-2023 |
| | | JP | 2023517567 A | 26-04-2023 |
| | | US | 2021282724 A1 | 16-09-2021 |
| | | WO | 2021180500 A1 | 16-09-2021 |

EPO FORM P0459

For more details about this annex : see Official Journal of the European Patent Office, No. 12/82